# EUROPEAN PATENT SPECIFICATION

(11) **EP 4 255 875 B1**
(45) Date of publication and mention of the grant of the patent: **02.10.2024**
(21) Application number: 21816484.6
(22) Date of filing: 01.12.2021
(51) Int. Cl.: C07C 29/70, C07C 31/30, C07C 41/26, C07C 43/13

(54) **PROCESS FOR PREPARING METAL ALKOXIDES BY TRANSALCOHOLISATION**
VERFAHREN ZUR HERSTELLUNG VON METALLALKOXIDEN DURCH TRANSALKOHOLISIERUNG
PROCÉDÉ DE PRÉPARATION D'ALCOXYDES MÉTALLIQUES PAR TRANSALCOOLISATION

(30) Priority: 04.12.2020 EP 20211888
(43) Date of publication of application: 11.10.2023
(73) Proprietor: BASF SE, 67056 Ludwigshafen am Rhein (DE)
(72) Inventor: WEISSKER, Wolf-Steffen, 67056 Ludwigshafen (DE); RICHMOND, Edward, 67056 Ludwigshafen (DE)
(86) International application number: PCT/EP2021/083700
(87) International publication number: WO 2022/117612

(56) References cited:
- WO-A1-2015/113518
- US-A- 3 418 383

## Description

The present invention relates to a process for preparing metal alkoxides by reacting lower metal alkoxides with higher alcohols in the presence of an auxiliary alcohol.

Metal alkoxides are useful as strong bases in the synthesis of numerous compounds, for example in the production of active ingredients for agricultural or pharmaceutical applications. Metal alkoxides may also be used as catalysts for base-catalyzed reactions such as transesterification or amidation reactions, or as anionic polymerization initiators.

Metal alkoxides (MOR) are obtainable by reacting metal hydroxides (MOH) with alcohols (ROH) in a reactive distillation column, as indicated in the following equation. Water obtained in the reaction is removed with the distillate and the reaction is driven towards the synthesis of MOR.

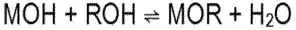

EP 0 091 425 A1 describes such a process, wherein an aqueous solution of an alkali metal hydroxide is fed into the rectifying section of a reactive distillation column, and gaseous alcohol is fed into the bottom of the column. Water is removed via the head of the column as an azeotrope, which is subsequently separated. Similar processes are described in EP 1 997 794 A1 and DD 246 988 A1.

Metal alkoxides are also obtainable by transalcoholisation of metal alkoxides of lower alcohols (MOR¹) with higher alcohols (R²OH) via reactive distillation to obtain higher metal alkoxides (MOR²) and lower alcohols (R¹OH), as indicated in the following equation.

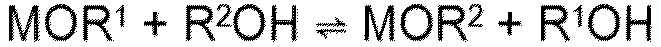

Such a process is described, e.g., in DE 27 26 491 A1 and DE 1 254 612 B. A mixture of a higher alcohol and a lower metal alkoxide is fed into a reactive distillation column.

The known processes for the production of metal alkoxides of high-boiling alcohols, such as C₁₀-alcohols, have high energetic requirements. Moreover, transalcoholisation processes may suffer from a poor solubility of the lower metal alkoxide in the higher alcohol, leading to an undesired precipitation of the lower metal alkoxide in the column. The solid deposits may cause clogging of the column, and their removal requires interrupting the reactive distillation and emptying the column, which is a time- and cost-intensive process.

Such precipitation especially occurs when the higher alcohol and the lower alcohol show a large degree of structural dissimilarity, e.g., a large difference in carbon number. Metal methoxides and ethoxides are readily available as inexpensive lower metal alkoxide starting materials, and the choice of other metal alkoxides (having a carbon number closer to that of the higher alcohol) is not a viable option from an economic point of view.

It is an object of the invention to provide a process for preparing metal alkoxides in a reactive distillation column which enables high conversion of a lower metal alkoxide to a higher metal alkoxide while limiting the formation of solid deposits in the column. The process should have low energy requirements.

The present invention provides a process for preparing metal alkoxides by transalcoholisation, comprising:
- feeding a lower metal alkoxide and a higher alcohol into a reactive distillation column;
- removing a solution of a higher metal alkoxide in the higher alcohol from the bottom of the column or from a recycle stream taken from the bottom of the column; and
- removing a gaseous lower alcohol from the top of the column, at least partially condensing the gaseous lower alcohol, and recirculating a part of the condensate to the top of the column;
wherein an auxiliary alcohol is provided in the reactive distillation column, the boiling point of the auxiliary alcohol being between the boiling point of the lower alcohol and the boiling point of the higher alcohol at the pressure prevailing in the reactive distillation column.

The lower metal alkoxide, the auxiliary alcohol and the higher alcohol are brought into contact with each other in a reactive distillation column. In the context of the present invention, the term "reactive distillation column" and the term "column" are used interchangeably.

The terms "lower" and "higher" alcohol refer to alcohols having lower and higher boiling temperatures, relative to each other. In other words, the higher alcohol ("transalcoholisation alcohol") has a higher boiling point than the alcohol of the lower metal alkoxide at the pressure prevailing in the reactive distillation column. The boiling point of the auxiliary alcohol is between the boiling point of the lower alcohol and the boiling point of the higher alcohol at the pressure prevailing in the reactive distillation column. The process according to the invention is generally applicable to the reaction of metal alkoxides of lower alcohols with higher alcohols in the presence of auxiliary alcohols, provided that the alcohols are distillable at the pressure prevailing in the reactive distillation column.

Similarily, the term "lower metal alkoxide" refers to a metal alkoxide having an anion formed by abstraction of a proton from a lower alcohol as defined above; the term "higher metal alkoxide" refers to a metal alkoxide having an anion formed by abstraction of a proton from a higher alcohol as defined above; and the term "auxiliary metal alkoxide" refers to a metal alkoxide having an anion formed by abstraction of a proton from an auxiliary alcohol as defined above.

The lower metal alkoxide, the auxiliary alcohol and the higher alcohol are brought into contact with each other in a reactive distillation column. In the lower part of the column, the higher alcohol and the auxiliary alcohol are partially gaseous and rise towards the top of the column, while a solution of the lower metal alkoxide trickles towards the bottom of the column. The trickling solution of the lower metal alkoxide (MOR¹) reacts with auxiliary alcohol (R^{X}OH) rising in countercurrent to form the lower alcohol (R¹OH) and an auxiliary metal alkoxide (MOR^{X}), as shown in the following equation.

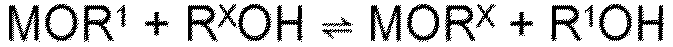

The transalcoholisation reaction proceeds preferentially in the liquid phase. Removal of the lower alcohol (R¹OH) formed is the driving force of the reaction. Gaseous lower alcohol rises towards the top of the column, while a solution of the auxiliary metal alkoxide trickles towards the bottom of the column. The trickling solution of the auxiliary metal alkoxide (MOR^{X}) reacts with higher alcohol (R²OH) rising in countercurrent to form the higher metal alkoxide (MOR²) and the auxiliary alcohol (R^{X}OH), as shown in the following equation.

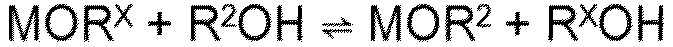

Since the solubility of the lower metal alkoxide in the auxiliary alcohol is generally higher in comparison to the solubility of the lower metal alkoxide in the higher alcohol, the auxiliary alcohol compatibilizes the lower metal alkoxide and the higher alcohol. Thus, the formation of solid deposits in the column is largely reduced or prevented altogether. It is moreover advantageous for the auxiliary metal alkoxide to exhibit a relatively high solubility in the higher alcohol. Notably, the auxiliary alcohol is not consumed in the process according to the invention and essentially remains in the reactive distillation column.

Gaseous lower alcohol is withdrawn at the top of the reactive distillation column and condensed, and a solution of the higher metal alkoxide in the higher alcohol is removed from the bottom of the column or from a recycle stream taken from the bottom of the column. Thus, a top fraction and a bottom fraction are obtained. The purity of these fractions is decisive for the efficiency of the process and the usefulness of the fractions.

Advantageously, the inventive process allows for a high purity of both the lower alcohol obtained at the top of the column and of the solution of the higher metal alkoxide obtained at the bottom of the column. In other words, the gaseous lower alcohol advantageously comprises only low or negligible amounts of higher alcohol and auxiliary alcohol, and the solution of the higher metal alkoxide comprises only low or negligible amounts of lower alcohol, lower metal alkoxide, auxiliary alcohol and auxiliary metal alkoxide.

The gaseous lower alcohol removed at the top of the column is at least partially condensed, and a part of the condensate obtained is recirculated to the top of the column as reflux. In the column, a gas phase and a liquid phase are countercurrently in contact with each other. A mass transfer takes place between the gas phase and the liquid phase. The more volatile components accumulate in the gas phase towards the top of the column, while and the less volatile components accumulate in the liquid phase towards the bottom of the column.

The purity of the obtained fractions, especially of the solution of the higher metal alkoxide removed from the bottom of the column or from a recycle stream taken from the bottom of the column, may be adjusted, e.g., by the reflux ratio at the top of the column. The reflux ratio is the ratio of the amount of condensate returned to the column (reflux) and the amount of condensate removed from the process. Generally, a higher reflux ratio results in a higher purity of the solution of the higher metal alkoxide. However, a higher reflux ratio results in higher energy requirements of the process. The process of the invention advantageously allows for a relatively low reflux ratio while achieving a sufficiently high purity of the gaseous lower alcohol.

The reaction equilibrium constant of the reactions described above are dependent on the acidity of the educt alcohol. For primary alcohols with short alkyl chains, the reaction equilibrium is further on the product side compared to the reaction equilibrium for longer chain secondary or tertiary alcohols, which is far on the side of the educt alcohol and metal alkoxide. By appropriate choice of the auxiliary alcohol, the reaction equilibrium constants of the two reactions decribed are in a range which allows an economically low reflux ratio.

The lower metal alkoxide may be a metal alkoxide of a primary monohydric C₁-C₃-alcohol, i.e. methoxide, ethoxide or propoxide. Preferably, the lower metal alkoxide is a metal methoxide or metal ethoxide, in particular metal methoxide.

The lower metal alkoxide is preferably an alkali metal alkoxide, more preferably a sodium alkoxide or a potassium alkoxide.

In a particularly preferred embodiment, the lower metal alkoxide is sodium methoxide or potassium methoxide, in particular sodium methoxide.

The higher alcohol may be selected from linear, branched or cyclic monohydric C₆-C₁₆-alcohols, such as hexanol, heptanol, octanol, nonanol, decanol, undecanol, dodecanol, tetradecanol, hexadecanol and their constitutional isomers, including cyclic alcohols and the primary, secondary and tertiary forms of the alcohols. In principle, polyhydric C₂-C₁₀-alcohols such as diols, for example ethylene glycol, diethylene glycol, propanediol or butanediol, and their constitutional isomers, can also be used.

The carbon chain of the higher alcohols may be interrupted by one or more oxygen atoms, at least two carbon atoms being between the interrupting oxygen atoms and between the interrupting oxygen atoms and each hydroxy group included in the compound. An example of a monohydric alcohol whose carbon chain is interrupted by an oxygen atom is 3-methyl-3-methoxybutanol or 1-methoxy-2-propanol.

The higher alcohol may also comprise one or more substituents which are inert under the conditions of the inventive process, i.e. which do not react with the compounds or intermediates present, such as -F or -Cl.

The higher alcohol is preferably selected from 3-methyl-3-methoxybutanol, 2-methyl-2-pentanol, 3-methyl-3-pentanol, 3-ethyl-3-pentanol, 2-methyl-2-hexanol, 3-methyl-3-hexanol, 3,7-dimethyl-3-octanol (tetrahydrolinalool, THL), 3,7-dimethyl-1-octanol, and 2,6-dimethyl-2-octanol (tetrahydromyrcenol). 3,7-Dimethyl-3-octanol is particularly preferred.

Preferably, the higher metal alkoxide obtained from the higher alcohol is liquid at STP (20 °C and 1 bar absolute). For example, sodium tetrahydrolinaloolate is liquid at STP. Due to the enhanced stability as a liquid, precipitation of higher metal alkoxide can be more reliably avoided across the length of the reactive distillation column. Also, the product metal alkoxide can be withdrawn as a highly concentrated solution.

The auxiliary alcohol preferably has a boiling point at least 10 °C, more preferably at least 15 °C, most preferably at least 20 °C above the boiling point of the lower alcohol at the pressure prevailing in the reactive distillation column. Preferably, the boiling point of the auxiliary alcohol is at least 10 °C, more preferably at least 15 °C, most preferably at least 20 °C below the boiling point of the higher alcohol at the pressure prevailing in the reactive distillation column.

The auxiliary alcohol may be selected from monohydric C₃-C₇-alcohols, i.e. propanol, butanol, pentanol, hexanol, heptanol and their constitutional isomers, including cyclic alcohols and the primary, secondary and tertiary forms of the alcohols. The carbon chain of the alcohols may be interrupted by one or more oxygen atoms, at least two carbon atoms being between the interrupting oxygen atoms and between the interrupting oxygen atoms and each hydroxy group included in the compound. An example of a monohydric alcohol whose carbon chain is interrupted by an oxygen atom is 1-methoxy-2-propanol.

The auxiliary alcohol may also comprise one or more substituents which are inert under the conditions of the process according to the invention, i.e. which do not react with the compounds or intermediates present.

Suitable auxiliary alcohols include 1-methoxy-2-propanol, 2-butanol, 2-methyl-2-butanol, 3-methyl-2-butanol, tert-butanol, 3-methyl-3-pentanol and 3-ethyl-3-pentanol. 2-butanol, 1-methoxy-2-propanol, and 3-methyl-2-butanol are particularly preferred, especially 3-methyl-2-butanol.

Notably, the process of the invention relates to a range of possible combinations of metal alkoxides and alcohols wherein, e.g., an alcohol may in one specific embodiment constitute the higher alcohol, and in another specific embodiment constitute the auxiliary alcohol. It is understood that the specific examples of the lower metal alkoxide, higher alcohol and auxiliary alcohol provided above are illustrative, without restricting the possibility of an alcohol constituting, e.g., either an auxiliary alcohol or a higher alcohol, depending on the differences in boiling points of the components used in a specific embodiment.

The purity of the obtained gaseous and liquid fractions and/or the energy requirements of the process may be influenced, e.g., by the location of the feed of the lower metal alkoxide and the feed of the higher alcohol.

The lower metal alkoxide may be fed into the reactive distillation column via a side feed. The term "side feed" means that the location of the feed is below the top of the column and above bottom of the column. Preferably, the lower metal alkoxide is fed into the middle or upper half of the column. The location of the feed of the lower metal alkoxide defines a rectifying section (above the location of the feed) and a stripping section (below the location of the feed). The transalcoholisation takes place in the stripping section of the column.

Generally, the lower metal alkoxide is fed into the column as a solution, for example via a pump. For example, the lower metal alkoxide may be fed into the column as a solution in the lower alcohol, the solution comprising 20 to 40% by weight of the lower metal alkoxide, preferably 25 to 35% by weight of the lower metal alkoxide, such as 28 to 32% by weight of the lower metal alkoxide, relative to the total weight of the solution of the lower metal alkoxide. Percentages are herein provided as percent by weight, unless noted otherwise.

The feed inlet temperature of the solution of the lower metal alkoxide is preferably at least 6 °C, in particular at least 10 °C and particularly preferably at least 20 °C, for example 25 °C or 30 °C. The solution of the lower metal alkoxide can be preheated to boiling temperature, for example in a heat exchanger, in which the solution of the higher metal alkoxide removed from the bottom of the column or from a recycle stream taken from the bottom of the column is simultaneously cooled. This is particularly advantageous with regard to the energy requirements of the process.

The higher alcohol is preferably fed into the column at one or more points below the feed inlet of the lower metal alkoxide. The higher alcohol may, e.g., be fed into the stripping section, the bottom and/or the recycle stream taken from the bottom of the column. Preferably, the higher alcohol is fed into the bottom and/or the recycle stream taken from the bottom of the column. "A recycle stream taken from the bottom of the column" is understood to relate to a stream taken from the bottom of the column and recirculated to the column, preferably recirculated to the bottom of the column.

It was found that feeding the higher alcohol into the reactive distillation column below the feed of the lower metal alkoxide causes a significantly lower contamination of the solution of the higher metal alkoxide with lower alcohol. In order to achieve the desired specifications, a lower reflux ratio and a reduced bottoms circulation is therefore required in the process according to the invention. Significantly lower energy requirements may thus be achieved for the same purity specifications.

The higher alcohol may be fed into the column in liquid or gaseous form. The higher alcohol is preferably fed into the bottom of the column and/or the recycle stream taken from the bottom of the column in liquid form, especially into the recycle stream.

The auxiliary alcohol is provided in the reactive distillation column, typically prior to start-up of the reactive distillation column. The auxiliary alcohol is not consumed and essentially remains within the reactive distillation column, as may be monitored by analysis of the obtained gaseous and liquid fractions comprising only negligible amounts of auxiliary alcohol and auxiliary metal alkoxide.

Over an extended period of production, it may nonetheless be necessary to replenish auxiliary alcohol to the column in a batch-wise or continuous manner, preferably a continuous manner, so as to maintain an effective concentration of auxiliary alcohol in the column. Preferably, auxiliary alcohol is fed into the reactive distillation column via a side feed, in particular via a side feed located between the feed of the lower metal alkoxide and the feed of the higher alcohol. In a particularly preferred embodiment, the auxiliary alcohol is fed into the reactive distillation column well above the bottom of the column, e.g., lengthwise at at least 5% of the column height, more preferably at at least 15% of the column height, such as at at least 30% of the column height.

The reactive distillation column generally comprises a reboiler, preferably a circulation reboiler. In the reboiler, a partial stream of the solution of the higher-boiling metal alkoxide removed from the bottom of the column is heated and at least partially returned to the bottom of the column in gaseous form. Alternatively or additionally, the bottom of the column may be heated directly. Some of the higher alcohol is present in the reactive distillation column in gaseous form and rises towards the top of the column.

A solution of the higher metal alkoxide in the higher alcohol is removed from the bottom of the column and/or from the recycle stream taken from the bottom of the column. Advantageously, the solution of the higher metal alkoxide in the higher alcohol contains only small amounts of the lower alcohol, which allows for an efficient process.

Preferably, the solution of the higher metal alkoxide comprises at most 1% by weight of the lower alcohol, more preferably at most 0.1% by weight of the lower alcohol and most preferably at most 0.02% by weight of the lower alcohol, for example 0.005 to 0.02% by weight of the lower alcohol, relative to the total weight of the higher metal alkoxide solution. The concentration of the lower alcohol in the solution of the higher metal alkoxide can be determined, e.g., by means of vapor space analysis or gas chromatography.

Moreover, the solution of the higher metal alkoxide in the higher alcohol advantageously contains not more than trace amounts of the auxiliary alcohol. Preferably, the solution of the higher metal alkoxide comprises at most 1% by weight of the auxiliary alcohol, more preferably at most 0.1% by weight of the auxiliary alcohol and most preferably at most 0.02% by weight of the auxiliary alcohol, for example 0.005 to 0.02% by weight of the auxiliary alcohol, relative to the total weight of the higher metal alkoxide solution. The concentration of the auxiliary alcohol in the solution of the higher metal alkoxide can be determined, e.g., by means of gas chromatography.

The solution of the higher metal alkoxide usually comprises 3 to 90% by weight of the higher metal alkoxide, relative to the total weight of the solution of the higher metal alkoxide taken off. The amount of higher metal alkoxide in the withdrawn solution generally depends on the solubility of the higher metal alkoxide in the higher alcohol. For the sake of efficiency, the transalcoholisation process should be carried out so that the withdrawn solution of the higher metal alkoxide comprises as much of the product metal alkoxide as possible. The concentration of the higher metal alkoxide in the solution of the higher alcohol can be determined, e.g., by titration.

The top condensate preferably comprises at most 1% by weight of the higher alcohol, more preferably at most 0.1% by weight of the higher alcohol and most preferably at most 0.01 % by weight of the higher alcohol, relative to the total weight of the overhead condensate. The concentration of the higher alcohol in the top condensate may be determined, e.g., by means of gas chromatography.

The top condensate preferably comprises at most 5% by weight of the auxiliary alcohol, more preferably at most 1 % by weight of the auxiliary alcohol and most preferably at most 0.1 % by weight of the auxiliary alcohol, relative to the total weight of the overhead condensate. The concentration of the auxiliary alcohol in the top condensate may be determined, e.g., by means of gas chromatography.

The amounts of higher alcohol and auxiliary alcohol in the top condensate are representative of the purity of the obtained gaseous fraction.

The inventive process may be performed in any reactive distillation column as typically used. Suitable types of reactive distillation columns include packed columns, such as columns with random packing or structured packing, plate columns (i.e., tray columns), and mixed columns comprising both packings and trays.

Suitable plate columns may comprise internals over which the liquid phase flows. Suitable internals include sieve trays, bubble cap trays, valve trays, tunnel trays and Thormann^{®} trays, in particular bubble cap trays, valve trays tunnel trays and Thormann^{®} trays.

Random packed columns may be filled with a variety of shaped bodies. Heat and mass transfer are improved by enlarging the surface area by means of shaped bodies, which usually have a size in the range of 25 to 80 mm. Suitable shaped bodies include Raschig rings (hollow cylinders), Lessing rings, Pall rings, Hiflow rings and Intalox saddles. The packing materials may be provided in the column in a regular or irregular manner (as bulk material, i.e. loosely filled). Suitable materials include glass, ceramics, metal and plastics.

Structured packings are an advancement of regular packings and have a regularly shaped structure. This allows for the reduction of gas flow pressure loss. Suitable types of structured packings include fabric and metal sheet packings.

Preferably, both the rectifying section of the column and the stripping section of the column comprise trays. As discussed above, the auxiliary alcohol is preferably fed into the reactive distillation column well above the bottom of the column. In a practical embodiment, the auxiliary alcohol side feed is lengthwise at least 5 trays above the bottom of the column. For example, in a column comprising 80 trays, the metal methoxide may be fed onto the 40^{th} tray, the auxiliary alcohol may be fed onto the 38^{th} tray, and the reactant alcohol may be fed onto the 10^{th} tray.

The term "top" or "head" of the column refers to a region free of internals located above the topmost tray or above the topmost layer of packing. It is generally formed by a domed base (head, e.g., Klöpper head or Korbbogen head), which forms the terminating element of the reactive distillation column.

The term "bottom" or "sump" of the column refers to a region free of internals located below the lowest tray or lowest layer of packing.

The number of theoretical plates in the rectifying section depends on the difference between the vapor pressures of the auxiliary alcohol and the lower alcohol, whereby a higher number of theoretical plates is advantageous in case of a small difference. The number of theoretical plates in the stripping section depends on the difference between the vapor pressures of the auxiliary alcohol and the higher alcohol, as well as on the equilibrium position of the transalcoholisation reaction(s). A higher number of theoretical plates is advantageous when the equilibrium is predominantly on the side of the starting materials. The number of theoretical plates in both the rectifying section and the stripping section also depends on the desired purity of the bottom and top product and the reflux ratio used, whereby a higher number of theoretical plates is required to achieve a higher purity at a given reflux ratio.

The column can consist of a plurality of cascaded individual vessels or cascaded individual columns. The individual vessels and columns are preferably arranged one above the other, and may be laterally offset from one another. The individual vessels and columns may also be arranged side by side using suitable pumping elements.

In both cases it must be ensured by means of appropriate connecting lines and, optionally, transport means, that the rectifying section and the stripping section are coupled directly and that the reflux in the rectifying section advantageously effects the transalcoholisation taking place in the stripping section. Preferably, the stripping section and the rectifying section are arranged one above the other in a single column.

The gaseous lower alcohol removed from the top of the column is at least partially condensed to yield a top condensate. Condensation is preferably carried out in a plate heat exchanger, shell and tube heat exchanger or straight tube condenser, or several such condensers connected in series. Preference is given to using straight tube condensers or shell and tube heat exchangers or a combination thereof. Depending on the design, the condensers can be cooled, for example, by air, cooling water or brine.

A part of the condensate is recirculated to the top of the column as reflux, and the remainder of the condensate is removed from the process. The reflux ratio influences the purity of the obtained gaseous fraction and especially the obtained liquid fraction. The reflux ratio is the ratio of the amount of condensate (kg/h) returned to the column (reflux) and the amount of condensate (kg/h) removed from the process. The higher the reflux ratio, the higher the purity of the the obtained gaseous and liquid fractions.

The optimum reflux ratio depends primarily on the type of higher alcohol used in the inventive process, and should advantageously be determined in preliminary tests. The optimum reflux ratio may be determined in a known manner so that at an economic optimum with regard to the energy required to separate the lower, auxiliary and higher alcohol, an optimum is achieved with regard to the purity of the lower alcohol removed from the process and the solution of the higher metal alkoxide.

The lower alcohol removed from the process, i.e. the condensate removed from the process, is generally of high purity and can be repurposed without further distillation. Only the excess amount of the top condensate, which is not needed to adjust the reflux amount, is removed from the system. The remaining amount of the top condensate is returned to the top of the column as reflux.

The reactive distillation column generally comprises a sump circulation ("bottoms circulation"). Preferably, the sump circulation comprises a reboiler, preferably a circulation reboiler, as described above. In the reboiler, a partial stream of the solution of the higher-boiling metal alkoxide removed from the bottom of the column is heated and at least partially returned to the bottom of the column in gaseous form.

In one embodiment, a substream of the solution of the higher metal alkoxide in the higher alcohol removed from the bottom of the column is returned to the bottom of the column, while another substream of the solution of the higher metal alkoxide in the higher alcohol removed from the bottom of the column is removed from the process.

In another embodiment, a substream of the solution of the higher metal alkoxide in the higher alcohol removed at the bottom of the column is recycled to the bottom of the column, preferably via a reboiler, while another substream of the solution of the higher metal alkoxide in the higher alcohol withdrawn at the bottom of the column is removed from the process. In a further embodiment, the sump circulation comprises a reboiler, and a substream of the sump circulation stream is recycled to the bottom of the column, while another substream of the sump circulation stream is removed from the process, preferably after the reboiler.

The reactive distillation column typically comprises a reboiler integrated in the bottom of the column or, preferably, a reboiler comprised in the sump circulation. A substream of the solution of the higher metal alkoxide removed at the bottom of the column is fed to the reboiler via sump circulation and then returned to the column as a heated fluid stream, which may optionally comprise two phases. Suitable reboilers include evaporators, natural circulation reboilers, forced circulation reboilers and forced circulation flash reboilers.

A forced circulation reboiler uses a pump to circulate the liquid to be evaporated through the heater. The obtained vapor/liquid mixture is then returned to the column.

In forced circulation flash reboilers, a pump is also used to circulate the liquid to be evaporated through the heater. A superheated liquid is obtained and decompressed into the bottom of the column. The pressure to which the higher metal alkoxide solution recycled to the column is subjected is increased by superheating. The superheated recycle stream is decompressed via a flow limiter. Thus, the liquid is superheated to above its boiling point in relation to the pressure prevailing in the column.

When the superheated liquid passes through the flow limiter and re-enters the column, sudden evaporation of the liquid occurs. This sudden evaporation, which is accompanied by a considerable increase in volume, results in an acceleration of the fluid stream entering the column. Preferably, the flow limiter is located immediately before the point of re-entry of the superheated liquid into the column, or even within the column's interior.

Preferably, the flow limiter is selected from an orifice plate, a valve, a throttle, a perforated disc, a nozzle, a capillary, or combinations thereof. Preferably a valve, such as a rotary plug valve, is used as the flow limiter. In a preferred embodiment, the opening profile of the flow limiter is adjustable. This allows for maintaining the pressure in the reboiler above the boiling pressure of the liquid, relative to the pressure prevailing in the column, under varying flow velocities, which may occur, e.g., during start-up and shut-down processes.

Advantageously, operating the reboiler as a forced circulation reboiler or a forced flash circulation reboiler increases the flow velocity of the liquid in the heater, such as in the tube bundle of a heat exchanger, in comparison to a natural circulation reboiler. The higher flow velocity allows for an improved heat transfer between the heat exchanger and the heated fluid, which in turn helps to prevent local overheating.

The pump used in a forced circulation evaporator or a forced circulation flash evaporator is preferably arranged between the removal line and the evaporator.

In a preferred embodiment, the column comprises a forced circulation evaporator and the higher alcohol is fed into a stream supplied to the forced circulation reboiler in liquid form.

Alternatively or in addition to the bottom circulation reboiler, the bottom of the column can be heated directly, e.g., by an internal evaporator.

At a given pressure, the temperature of the bottom of the column determines the concentration of the higher metal alkoxide in the solution removed from the bottom of the column or from a recycle stream taken from the bottom of the column. The temperature and thus the concentration are suitably selected so that the higher metal alkoxide always remains in solution at the bottom of the column. The bottom temperature may be adjusted by means of a reboiler and/or direct heating of the bottom.

The inventive process may be performed at ambient pressure as well as at reduced or elevated pressure. The pressure prevailing in the column is preferably in the range of 0.2 to 10 bar absolute, more preferably in the range of 0.5 to 3 bar absolute, most preferably at ambient pressure, e.g.,1 bar absolute.

The equilibrium position of the transalcoholisation may be temperature-dependent for some alkoxides. In these cases, higher temperatures may affect higher turnover. It may also be advantageous to perform the inventive process under elevated pressure, for example at least 1.5 bar absolute, at least 2.5 bar absolute or at least 5.0 bar absolute.

The inventive process may be carried out continuously or batchwise. Preferably, the inventive process is carried out continuously.

Within the reactive distillation column, the reaction equilibrium is constantly readjusted due to the continuous mass transfer and the changing concentrations within the gas phase and the liquid phase, allowing for a high conversion rate. When the column is started up, the lower alcohol may be fed into the top of the column to adjust the amount of reflux, and the stripping section and the sump may be filled with the higher alcohol and the auxiliary alcohol. Lower alcohol can also be added to the higher alcohol and/or auxiliary alcohol.

After the operating temperature is reached, the solution of the lower metal alkoxide is added. Fresh lower alcohol is continuously formed during the reaction.

In a further embodiment, the reactive distillation column is filled with the auxiliary alcohol and the higher alcohol before start-up, with the auxiliary alcohol initially used as reflux. After the operating temperature is reached, the solution of the lower metal alkoxide is added. The addition of the lower metal alkoxide affects the temperature in the column, so that the final operating temperature is reached at a later point of time, especially in the column bottom.

The gaseous lower alcohol drawn off at the top of the column typically consists of essentially pure lower alcohol. The top condensate may be removed from the process without further purification and can be used, for example, for the production of lower metal alkoxides or solutions thereof. Optionally, the top condensate may be subjected to further work-up steps, such as distillation.

The solution of the higher metal alkoxide removed from the bottom of the column or from a recycle stream taken from the bottom of the column typically consists essentially of the higher alcohol and the higher metal alkoxide. The solution of the higher metal alkoxide can thus be used further as such, if necessary after cooling in a heat exchanger.

Alternatively, the higher metal alkoxide may be isolated from the solution in the higher alcohol according to methods known in the art, e.g., by evaporation of the higher alcohol.

The invention is further illustrated by the enclosed figure and the following examples.

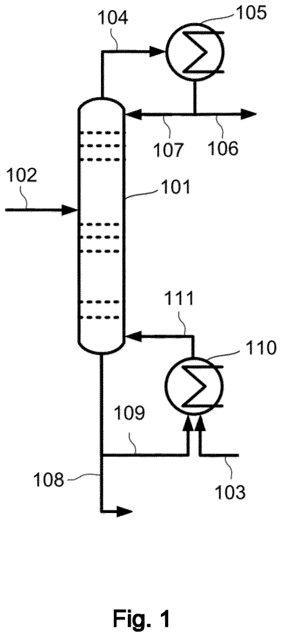

Fig. 1 shows a plant suitable for the production of metal alkoxides by the process of the invention.

According to Fig. 1, the plant comprises a reactive distillation column 101, in which an auxiliary alcohol is provided. A solution of a lower metal alkoxide is fed into column 101 via line 102. A higher alcohol is fed to the bottom of column 101 via line 103, reboiler 110 and line 111. The auxiliary alcohol is typically provided prior to start-up of column 101 via a line located between lines 102 and 111 (not shown in Fig. 1), and auxiliary alcohol is continuously fed to column 101 via said line in the course of the reaction to maintain an effective concentration of auxiliary alcohol in column 101.

At the top of column 101, a gaseous lower alcohol is removed via line 104 and condensed in condenser 105. A first stream of the condensed lower alcohol is removed from the process via line 106, while a second stream of the condensed lower alcohol is returned to the top of column 101 via line 107.

A solution of a higher metal alkoxide in the higher alcohol is removed from the bottom of the column. A first stream of the solution of the higher metal alkoxide is removed from the process via line 108, while a second stream of the solution of the higher metal alkoxide is returned to the bottom of the column via line 109 and reboiler 110 together with the higher alcohol from line 103 via line 111.

### Examples and Methods

Experiments were carried out on the production of sodium tetrahydrolinaloolate by transalcoholisation from sodium methoxide and tetrahydrolinalool (THL), using 2-butanol, 1-methoxy-2-propanol and 3-methyl-2-butanol as auxiliary alcohols.

### Methods

### A. Determination of Higher Alcohol and Auxiliary Alcohol in the Top Condensate

A sample of the top condensate was taken, 1,4-dioxane was added as internal standard and the sample was analyzed for its THL and auxiliary alcohol content by gas chromatography (separation column RTX-5 Amine, length 30 m, internal diameter 0.32 mm, film thickness 1.5 µm, TCD detector). The detection limit was approximately 500 mg/kg.

### B. Determination of Lower Alcohol and Auxiliary Alcohol in the Bottom Output

### B.1 Auxiliary Alcohol: 2-Butanol

500 mg of a sample of the solution of the product metal alkoxide were taken and allowed to cool to room temperature (approximately 23 °C). The sample was mixed with about 0.5 mg 2-methyl-2-butanol (as internal standard) in 1 mL ethanol, and 4 mL ethanol (without internal standard) were added to obtain a diluted sample. In cases where the sample was solid, it was melted at 60 °C before mixing with 2-methyl-2-butanol and ethanol.

The diluted sample was analyzed by gas chromatography (separation column DB-1, length 30 m, inner diameter 0.25 mm, film thickness 1.0 µm). Quantification was carried out using the standard addition method. The detection limit was 200 mg/kg.

### B.2 Auxiliary Alcohol: 1-Methoxy-2-Propanol

500 mg of a sample of the solution of the product metal alkoxide were taken and allowed to cool to room temperature (approximately 23 °C). The sample was mixed with about 0.5 mg sec-butanol (as internal standard) in 1 mL ethanol, and 4 mL ethanol (without internal standard) were added to obtain a diluted sample. In cases where the sample was solid, it was melted at 60 °C before mixing with sec-butanol and ethanol.

The diluted sample was analyzed by gas chromatography (separation column DB-1, length 30 m, inner diameter 0.25 mm, film thickness 1.0 µm). Quantification was carried out using the standard addition method. The detection limit was 200 mg/kg.

### B.3 Auxiliary Alcohol: 3-Methyl-2-Butanol

500 mg of a sample of the solution of the product metal alkoxide were taken and allowed to cool to room temperature (approximately 23 °C). The sample was mixed with about 0.5 mg n-hexane (as internal standard) in 1 mL isopropanol, a drop of phosphoric acid was added, and 3 mL isopropanol (without internal standard) were added to obtain a diluted sample. In cases where the sample was solid, it was melted at 60 °C before mixing with hexane, phosphoric acid and ethanol.

The diluted sample was analyzed by gas chromatography (separation column DB-1, length 30 m, inner diameter 0.25 mm, film thickness 1.0 µm). Quantification was carried out using the standard addition method. The detection limit was 200 mg/kg.

In the standard addition method, a multiple determination of the sample is performed, for example a double determination. A specific amount of the substance to be determined (the reactant alcohol) is added to each sample several times and the sample is measured after each addition. The increase of the substance is determined. The concentration of the reactant alcohol in the original sample can be calculated by linear regression.

The solubility of the samples must be checked in advance. If two phases are formed, the weight of the sample must be reduced.

### C. Determination of the Concentration of Sodium Alkoxide Tetrahydrolinaloolate in the Bottom Output

To determine the amount of sodium alkoxide tetrahydrolinaloolate in the bottom of the column, a sample was taken and the total content of bases consisting of alkoxide, hydroxides and carbonate was determined by titration in 2-propanol with trifluoromethanesulfonic acid (0.1 mol/l in 2 propanol). The amount of hydroxides and carbonates was determined by volumetric Karl Fischer titration (KFT), since these components react with the KF components in the KFT and form water. The contribution of hydroxides and carbonates were subtracted from the total base content to determine the alkoxide content.

### Examples

The examples were carried out in a plant according to Fig. 1, comprising a reactive distillation column with 80 bubble cap trays made of glass and a forced circulation flash reboiler. Table 1 shows the specific parameters of the examples.

The reboiler was heated with a commercial thermostat (Julabo HT6) with a maximum heating power of 5700 W. The diameter of the column was 50 mm. To avoid heat loss, the column was heated isothermally with an electrical protective heating system.

Prior to start-up, the column was filled with auxiliary alcohol and reactant alcohol. When the operating temperature was reached, sodium methoxide and reactant alcohol were fed to the column.

Sodium methoxide (30% by weight in methanol) was fed into the column from the side (tray 40 or 60). Tetrahydrolinalool (THL) was fed into the column before the reboiler or to tray 30.

The amount of product metal alkoxide, auxiliary alcohol and methanol in the solution of the product metal alkoxide removed at the bottom of the column ("bottom output") was determined.

At the top of the column, gaseous methanol was removed and condensed in a condenser. The amount of reactant alcohol and auxiliary alcohol in the top condensate was determined.

**Table 1**

| | Example 1 | Example 2 | Example 3 |
|---|---|---|---|
| auxiliary alcohol | 2-butanol | 1-methoxy-2-propanol | 3-methyl-2-butanol |
| amount of auxiliary alcohol in column [kg] | 0.221 | 0.417 | 0.459 |
| auxiliary alcohol feed [kg/h] | 0.009 | 0.009 | 0 |
| location of auxiliary alcohol feed | tray 40 | tray 40 | tray 38 |
| sodium methoxide feed [kg/h] | 0.08 | 0.08 | 0.25 |
| location of sodium methoxide feed | tray 60 | tray 60 | tray 40 |
| THL feed [kg/h] | 0.358 | 0.138 | 0.266 |
| location of TH L feed | tray 30 | tray 30 | reboiler |
| stream removed at top of column [kg/h] | 0.08 | 0.071 | 0.214 |
| stream removed at bottom of column [kg/h] | 0.375 | 0.165 | 0.307 |
| bottom circulation [kg/h] | 150 | 150 | 150 |
| reflux [kg/h] | 0.4 | 0.58 | 0.2 |
| reflux ratio | 5 | 8.2 | 0.935 |
| ratio of sodium methoxide feed to reflux | 0.2 | 0.138 | 1.25 |
| T (column head) [°C] | 59.9 | 60 | 46.8 |
| T (column bottom) [°C] | 188.3 | 194.6 | 206.5 |
| T (sodium methoxide feed) [°C] | 41.4 | 39.6 | 43.8 |
| pressure (column head) [mbar, absolute] | 698 | 697 | 499 |
| differential pressure of column [mbar] | 86.2 | 92.7 | 67.9 |
| sodium tetrahydrolinaloolate in bottom output [wt.-%] | 24.8 | 49 | 76.4 |
| methanol in bottom output [wt.-%] | <0.02 | 0.02 | <0.02 |
| auxiliary alcohol in bottom output [wt.-%] | <0.02 | 0.11 | <0.01 |
| THL in top condensate [wt.-%] | 0 | 0 | 0 |
| auxiliary alcohol in top condensate [wt.-%] | 15 | 8 | 0 |

In a comparative Example 4 carried out in a similar way to Example 2, but without auxiliary alcohol, a stable operation was not possible, as solid deposits formed in the column within 1 to 2 h and clogged the trays of the column.

It is evident that the presence of an auxiliary alcohol allows for a stable operation of the column. Moreover, the solution of sodium tetrahydrolinaloolate in tetrahydrolinalool is essentially free of both methanol and the auxiliary alcohol.

## Claims

1. A process for preparing metal alkoxides by transalcoholisation, comprising:
- feeding a lower metal alkoxide and a higher alcohol into a reactive distillation column;
- removing a solution of a higher metal alkoxide in the higher alcohol from the bottom of the column or from a recycle stream taken from the bottom of the column; and
- removing a gaseous lower alcohol from the top of the column, at least partially condensing the gaseous lower alcohol, and recirculating a part of the condensate to the top of the column;
wherein an auxiliary alcohol is provided in the reactive distillation column, the boiling point of the auxiliary alcohol being between the boiling point of the lower alcohol and the boiling point of the higher alcohol at the pressure prevailing in the reactive distillation column.

2. The process according to claim 1, wherein the lower alkoxide is fed into the reactive distillation column via a side feed.

3. The process according to claim 1 or 2, wherein the higher alcohol is fed into the bottom of the column and/or the recycle stream taken from the bottom of the column, preferably in liquid form.

4. The process according to any one of the preceding claims, wherein the solution of the higher metal alkoxide comprises at most 1% by weight of the lower alcohol, relative to the total weight of the solution of the higher metal alkoxide.

5. The process according to any one of the preceding claims, wherein the higher metal alkoxide solution comprises 3 to 90% by weight of the higher metal alkoxide, relative to the total weight of the higher metal alkoxide solution.

6. The process according to any one of the preceding claims, wherein the column comprises a forced circulation reboiler and the higher alcohol is fed into a stream supplied to the forced circulation reboiler in liquid form.

7. The process according to any one of the preceding claims, wherein the condensate of the gaseous lower alcohol comprises at most 1% by weight of the higher alcohol, relative to the total weight of the condensate.

8. The process according to any one of the preceding claims, wherein the lower metal alkoxide is fed into the column as a solution in the lower alcohol and the solution comprises 20 to 40% by weight of the lower metal alkoxide, relative to the total weight of the solution of the lower metal alkoxide.

9. The process according to any one of the preceding claims, wherein the lower metal alkoxide is an alkali metal alkoxide.

10. The process according to claim 9, wherein the lower metal alkoxide is a sodium alkoxide or a potassium alkoxide, preferably sodium methoxide or potassium methoxide, most preferably sodium methoxide.

11. The process according to any one of the preceding claims, wherein the higher alcohol is selected from 3-methyl-3-methoxybutanol, 2-methyl-2-pentanol, 3-methyl-3-pentanol, 3-ethyl-3-pentanol, 2-methyl-2-hexanol, 3-methyl-3-hexanol, 3,7-dimethyl-3-octanol, in particular 3,7-dimethyl-3-octanol.

12. The process according to any one of the preceding claims, wherein the boiling point of the auxiliary alcohol is at least 10 °C above the boiling point of the lower alcohol at the pressure prevailing in the reactive distillation column.

13. The process according to any one of the preceding claims, wherein the boiling point of the auxiliary alcohol is at least 10 °C below the boiling point of the higher alcohol at the pressure prevailing in the reactive distillation column.

14. The process according to any one of the preceding claims, wherein the auxiliary alcohol is selected from 1-methoxy-2-propanol, 2-butanol, 2-methyl-2-butanol, 3-methyl-2-butanol, tert-butanol, in particular 2-butanol, 1-methoxy-2-propanol, and 3-methyl-2-butanol.

15. The process according to any one of the preceding claims, wherein the higher metal alkoxide is liquid at a temperature of 20 °C and a pressure of 1 bar absolute.

## Patentansprüche

1. Verfahren zur Herstellung von Metallalkoholaten durch Umalkoholisierung, bei dem man
- ein niederes Metallalkoholat und einen höheren Alkohol in eine Reaktivdestillationskolonne einspeist;
- eine Lösung eines höheren Metallalkoholats in dem höheren Alkohol aus dem Sumpf der Kolonne oder aus einem aus dem Sumpf der Kolonne entnommenen Rückführstrom abzieht; und
- einen gasförmigen niederen Alkohol am Kopf der Kolonne abzieht, den gasförmigen niederen Alkohol zumindest teilweise kondensiert und einen Teil des Kondensats auf den Kopf der Kolonne zurückführt;
wobei in der Reaktivdestillationskolonne ein Hilfsalkohol bereitgestellt wird, wobei der Siedepunkt des Hilfsalkohols zwischen dem Siedepunkt des niederen Alkohols und dem Siedepunkt des höheren Alkohols bei dem in der Reaktivdestillationskolonne herrschenden Druck liegt.

2. Verfahren nach Anspruch 1, wobei das niedere Alkoholat über einen seitlichen Zulauf in die Reaktivdestillationskolonne eingespeist wird.

3. Verfahren nach Anspruch 1 oder 2, wobei der höhere Alkohol in den Sumpf der Kolonne und/oder den aus dem Sumpf der Kolonne entnommenen Rückführstrom eingespeist wird, vorzugsweise in flüssiger Form.

4. Verfahren nach einem der vorhergehenden Ansprüche, wobei die Lösung des höheren Metallalkoholats höchstens 1 Gew.-% des niederen Alkohols, bezogen auf das Gesamtgewicht der Lösung des höheren Metallalkoholats, umfasst.

5. Verfahren nach einem der vorhergehenden Ansprüche, wobei die Lösung des höheren Metallalkoholats 3 bis 90 Gew.-% des höheren Metallalkoholats, bezogen auf das Gesamtgewicht der Lösung des höheren Metallalkoholats, umfasst.

6. Verfahren nach einem der vorhergehenden Ansprüche, wobei die Kolonne einen Zwangsumlaufverdampfer aufweist und der höhere Alkohol in flüssiger Form in einen dem Zwangsumlaufverdampfer zugeführten Strom eingespeist wird.

7. Verfahren nach einem der vorhergehenden Ansprüche, wobei das Kondensat des gasförmigen niederen Alkohols höchstens 1 Gew.-% des höheren Alkohols, bezogen auf das Gesamtgewicht des Kondensats, umfasst.

8. Verfahren nach einem der vorhergehenden Ansprüche, wobei das niedere Metallalkoholat als Lösung in dem niederen Alkohol in die Kolonne eingespeist wird und die Lösung 20 bis 40 Gew.-% des niederen Metallalkoholats, bezogen auf das Gesamtgewicht der Lösung des niederen Metallalkoholats, umfasst.

9. Verfahren nach einem der vorhergehenden Ansprüche, wobei es sich bei dem niederen Metallalkoholat um ein Alkalimetallalkoholat handelt.

10. Verfahren nach Anspruch 9, wobei es sich bei dem niederen Metallalkoholat um ein Natriumalkoholat oder ein Kaliumalkoholat, vorzugsweise Natriummethanolat oder Kaliummethanolat, ganz besonders bevorzugt Natriummethanolat, handelt.

11. Verfahren nach einem der vorhergehenden Ansprüche, wobei der höhere Alkohol aus 3-Methyl-3-methoxybutanol, 2-Methyl-2-pentanol, 3-Methyl-3-pentanol, 3-Ethyl-3-pentanol, 2-Methyl-2-hexanol, 3-Methyl-3-hexanol, 3,7-Dimethyl-3-octanol, insbesondere 3,7-Dimethyl-3-octanol, ausgewählt wird.

12. Verfahren nach einem der vorhergehenden Ansprüche, wobei der Siedepunkt des Hilfsalkohols mindestens 10 °C über dem Siedepunkt des niederen Alkohols bei dem in der Reaktivdestillationskolonne herrschenden Druck liegt.

13. Verfahren nach einem der vorhergehenden Ansprüche, wobei der Siedepunkt des Hilfsalkohols mindestens 10 °C unter dem Siedepunkt des höheren Alkohols bei dem in der Reaktivdestillationskolonne herrschenden Druck liegt.

14. Verfahren nach einem der vorhergehenden Ansprüche, wobei der Hilfsalkohol aus 1-Methoxy-2-propanol, 2-Butanol, 2-Methyl-2-butanol, 3-Methyl-2-butanol, tert-Butanol, insbesondere 2-Butanol, 1-Methoxy-2-propanol und 3-Methyl-2-butanol, ausgewählt wird.

15. Verfahren nach einem der vorhergehenden Ansprüche, wobei das höhere Metallalkoholat bei einer Temperatur von 20 °C und einem Druck von 1 bar absolut flüssig ist.

## Revendications

1. Procédé pour la préparation d'alcoxydes métalliques par transalcoolisation, comprenant :
- l'alimentation d'un alcoxyde métallique inférieur et d'un alcool supérieur dans une colonne de distillation réactive ;
- l'élimination d'une solution d'un alcoxyde métallique supérieur dans l'alcool supérieur du fond de la colonne ou d'un flux de recyclage prélevé du fond de la colonne ; et
- l'élimination d'un alcool inférieur gazeux de la partie supérieure de la colonne, la condensation de manière au moins partielle de l'alcool inférieur gazeux, et la recirculation d'une partie du condensat vers la partie supérieure de la colonne ;
un alcool auxiliaire étant fourni dans la colonne de distillation réactive, le point d'ébullition de l'alcool auxiliaire étant entre le point d'ébullition de l'alcool inférieur et le point d'ébullition de l'alcool supérieur à la pression régnant dans la colonne de distillation réactive.

2. Procédé selon la revendication 1, l'alcoxyde inférieur étant alimenté dans la colonne de distillation réactive via une alimentation latérale.

3. Procédé selon la revendication 1 ou 2, l'alcool supérieur étant alimenté dans le fond de la colonne et/ou le flux de recyclage prélevé du fond de la colonne, préférablement sous forme liquide.

4. Procédé selon l'une quelconque des revendications précédentes, la solution de l'alcoxyde métallique supérieur comprenant au plus 1 % en poids de l'alcool inférieur, par rapport au poids total de la solution de l'alcoxyde métallique supérieur.

5. Procédé selon l'une quelconque des revendications précédentes, la solution d'alcoxyde métallique supérieur comprenant 3 à 90 % en poids de l'alcoxyde métallique supérieur, par rapport au poids total de la solution d'alcoxyde métallique supérieur.

6. Procédé selon l'une quelconque des revendications précédentes, la colonne comprenant un rebouilleur à circulation forcée et l'alcool supérieur étant alimenté dans un flux fourni au rebouilleur à circulation forcée sous forme liquide.

7. Procédé selon l'une quelconque des revendications précédentes, le condensat de l'alcool inférieur gazeux comprenant au plus 1 % en poids de l'alcool supérieur, par rapport au poids total du condensat.

8. Procédé selon l'une quelconque des revendications précédentes, l'alcoxyde métallique inférieur étant alimenté dans la colonne comme une solution dans l'alcool inférieur et la solution comprenant 20 à 40 % en poids de l'alcoxyde métallique inférieur, par rapport au poids total de la solution de l'alcoxyde métallique inférieur.

9. Procédé selon l'une quelconque des revendications précédentes, l'alcoxyde métallique inférieur étant un alcoxyde métallique alcalin.

10. Procédé selon la revendication 9, l'alcoxyde métallique inférieur étant un alcoxyde de sodium ou un alcoxyde de potassium, préférablement le méthoxyde de sodium ou le méthoxyde de potassium, le plus préférablement le méthoxyde de sodium.

11. Procédé selon l'une quelconque des revendications précédentes, l'alcool supérieur étant choisi parmi le 3-méthyl-3-méthoxybutanol, le 2-méthyl-2-pentanol, le 3-méthyl-3-pentanol, le 3-éthyl-3-pentanol, le 2-méthyl-2-hexanol, le 3-méthyl-3-hexanol, le 3,7-diméthyl-3-octanol, en particulier le 3,7-diméthyl-3-octanol.

12. Procédé selon l'une quelconque des revendications précédentes, le point d'ébullition de l'alcool auxiliaire étant au moins 10 °C au-dessus du point d'ébullition de l'alcool inférieur à la pression régnant dans la colonne de distillation réactive.

13. Procédé selon l'une quelconque des revendications précédentes, le point d'ébullition de l'alcool auxiliaire étant au moins 10 °C en dessous du point d'ébullition de l'alcool supérieur à la pression régnant dans la colonne de distillation réactive.

14. Procédé selon l'une quelconque des revendications précédentes, l'alcool auxiliaire étant choisi parmi le 1-méthoxy-2-propanol, le 2-butanol, le 2-méthyl-2-butanol, le 3-méthyl-2-butanol, le tert-butanol, en particulier le 2-butanol, le 1-méthoxy-2-propanol et le 3-méthyl-2-butanol.

15. Procédé selon l'une quelconque des revendications précédentes, l'alcoxyde métallique supérieur étant liquide à une température de 20 °C et une pression de 1 bar absolu.
